# EUROPEAN PATENT SPECIFICATION

(11) **EP 0 360 292 B1**
(45) Date of publication and mention of the grant of the patent: **05.08.1998**
(21) Application number: 89117589.5
(22) Date of filing: 22.09.1989
(51) Int. Cl.: A61K 7/50

(54) **Cleansing emulsions for beauty treatment**
Kosmetische Reinigungsmittel-Emulsionen
Emulsions nettoyantes pour le traitement esthétique

(30) Priority: 23.09.1988 IT 2205388
(43) Date of publication of application: 28.03.1990
(73) Proprietor: AUSIMONT S.p.A., I-20121 Milano (IT)
(72) Inventor: Pantini, Giovanni, Dr., I-20121 Milan (IT); Visca, Mario, Dr., I-15100 Alessandria (IT)
(74) Representative: Barz, Peter, Dr.

(56) References cited:
- DRUG & COSMETIC INDUSTRY, no. 9, 15th September 1988, pages 34-35,116,119; G. PANTINI et al.: "Perfluoropolyethers for cosmetics"

## Description

The present invention relates to cleansing emulsions for skin and hair. and the use thereof for shampoos, foam baths, liquid soaps and similar compositions.

An important problem in skin and hair cleansing is the effect of redistribution of sebum (the so-called "rebound" effect).

Several additives have been proposed in the past in order to reduce the sebum rebound effect. In particular, in US-A-3,972, 998 and 3,959,462 the use of film-forming fluorinated resins is disclosed. Unfortunately, the use of film-forming resins causes drawbacks: said resins are difficult to remove, can lead to accumulation phenomena and can cause toxicity problems.

It has now, surprisingly, been found that the effect of redistribution of sebum on skin and hair can be reduced without encountering the above problems, if cleansing compositions are used which contain perfluoro-polyethers having perfluoro-alkyl end groups.

It also has surprisingly been found that the use of the same compositions normalizes the redistribution of sebum on the skin of asteatosic subjects.

The use of perfluoropolyethers in cosmetics has been object of investigation, as pointed out in "Drug and Cosmetic Industry", no. 9, 15th Sept. 1988, pages 34-35, 116, 119. Said article discloses the formation of a thin film primarily having a barrier effect.

Object of the present invention is to provide cleansing emulsions for skin and hair which are capable of reducing the redistribution of sebum on skin and hair and of normalizing the redistribution of sebum on skin and hair of asteatosic subjects.

Accordingly, the present invention provides cleansing emulsions suitable for shampoos, foam baths, liquid soaps and similar compositions for skin and hair cleansing, capable of reducing the redistribution of sebum on skin and hair and of normalizing the redistribution of sebum on skin and hair of asteatosic subjects, said cleansing emulsions consisting of:
1) a perfluoropolyether with perfluoroalkyl end groups in an amount of from 0.01 to 20% by weight;
2) at least one surfactant having a low interface tension with said perfluoropolyether;
3) one or more thickening agents;
4) water;
5) optionally, one or more foam supporting substances;
6) optionally, one or more softeners and/or perfumes and/or dyes and/or preservatives and/or opacifiers and/or sequestering agents.

Said cleansing emulsions can be prepared by a process which shows the following steps:
(1) a perfluoropolyether containing perfluoroalkyl end groups as defined above and one or more surfactants, having a low interface tension with the perfluoropolyether are used;
(2) the surfactant(s) according to the above point (1) is (are) mixed with water and heated to a temperature which is sufficient for obtaining a homogeneous mixture or emulsion, the resulting mixture or emulsion being deaerated;
(3) thickeners and any foam-supporting substances are melted and are diluted with water until a medium-viscosity solution is obtained;
(4) the perfluoropolyether is added with strong stirring to the mixture according to the above point (2) or to the mixture according to the above point (3);
(5) the mixtures according to the above points (2) and (3) are mixed together and the obtained mixture is deaerated;
(6) any optional preservatives, dyes, perfumes, softeners, opacifiers and sequestering agents are added to the mixture according to the above point (2) and/or to the mixture according to the above point (3) and/or to the mixture according to the above point (5);
(7) the whole mixture is cooled with stirring.

The perfluoropolyethers having perfluoroalkyl end groups, i.e., without functional groups, are well-known products. Generally they are obtained as mixtures of compounds having a molecular weight within a certain range.

These perfluoropolyethers are described, together with corresponding methods of preparation, in various documents, e.g. GB-A-1,104,482; US-A-3,242,218; 3,665,041; 3,715,378; and 4,523, 039; and EP-A-148,482 and 191,490.

Examples of suitable perfluoropolyethers are those which are characterized by the presence of one or more perfluoro-oxyalkylene units selected from:
a) (CF₂-CF₂O);
b) (CF₂O);
c) (C₃F₆O), a simplified formula for 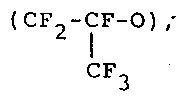
d) (CF₂O―CF₂―CF₂O);
e) (CF₂―CF₂-CF₂O);
f) 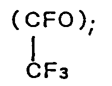 and
g) 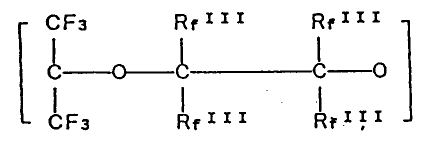 wherein the groups R_{f}III, the same or different from each other, are fluorine atoms or (preferably C₁₋₃-) perfluoroalkyl groups (e.g. CF₃, C₂F₅ and C₃F₇).

Perfluoropolyethers suitable for use in the present invention preferably contain one or more of the following individual perfluorooxyalkylene units or combinations of perfluorooxyalkylene units:
(I) (CF₂-CF₂O) and (CF₂O), said units being randomly distributed along the perfluoropolyether chain;
(II) 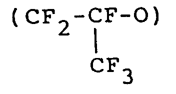 and (CFXO), wherein X is F or CF₃, said units being randomly distributed along the perfluoropolyether chain;
(III) (CF₂-CF₂O), 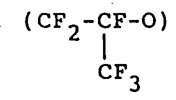 and (CFXO), wherein X is F or CF₃, said units being randomly distributed along the perfluoropolyether chain;
(IV) 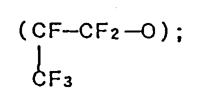
(V) (CF₂―CF₂―CF₂O);
(VI) (CF₂―CF₂O);
(VII) 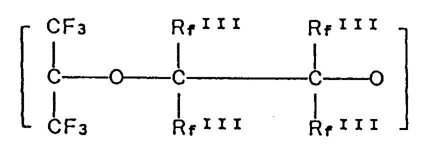 wherein the groups R_{f}III, the same or different from each other, are fluorine atoms or (preferably C₁₋₃-) perfluoroalkyl groups; and
(VIII) (CF₂O-CF₂-CF₂O).

Also suitable are perfluoropolyethers which contain perfluorooxetane rings of formula 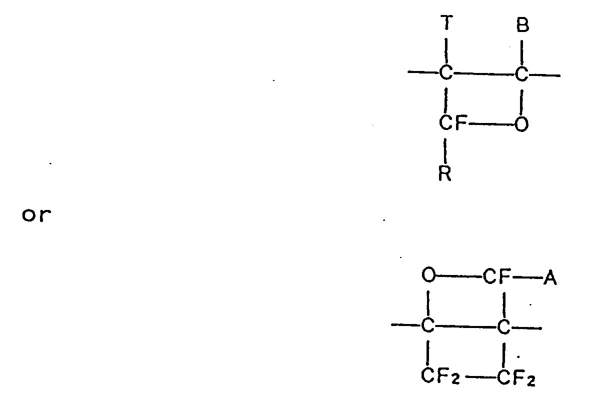 wherein T, B and R, the same or different from each other, are perfluorooxyalkyl, perfluoropolyoxyalkyl or perfluoroalkyl radicals, and
A is a perfluorooxyalkyl, perfluoropolyoxyalkyl or perfluoroalkyl radical.

Examples of perfluoroalkyl radicals are CF₃, C₂F₅ and C₃F₇ while examplary perfluorooxyalkyl radicals are those given above under (a) to (g), terminated with a F atom. Suitable perfluoropolyoxyalkyl radicals include those composed of one or more units given above under (a) to (g), also terminated by a F atom.

Examples of suitable perfluoropolyethers containing perfluorooxyalkylene units are those belonging to the following classes:
A) 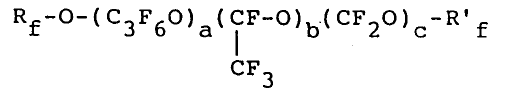 wherein:
   - R_{f} and R'_{f},: the same or different from each other, are selected from CF₃, C₂F₅ and C₃F₇; the units in brackets are randomly distributed along the polyether chain;
   - a: is an integer; and
   - b and c: represent integers or zero; the ratio ${\text{a}}_{̲} \text{/(} {\text{b}}_{̲} \text{+} {\text{c}}_{̲} \text{)}$ ranging from 5 to 40 when the sum (b+c) is different from zero;
B) CF₃O-(C₂F₄O)_{d}(CF₂O)ₑ-CF₃
   wherein the C₂F₄O and CF₂O units are randomly distributed along the polyether chain; d and e are integers; and the ratio d/e ranges from 0.3 to 5;
C) CF₃O-(C₃F₆O)_{f}(C₂F₄O)_{g}(CFXO)ₕ-CF₃
   wherein the C₃F₆O, C₂F₄O and CFXO units are randomly distributed along the polyether chain;
   X is F or CF₃;
   f, g and h are integers;
   the ratio ${\text{f}}_{̲} \text{/(} {\text{g}}_{̲} \text{+} {\text{h}}_{̲}$) ranges from 1 to 50; and
   the ratio g/h ranges from 1 to 10;
D) R³_{f}O-(CF₂CF₂CF₂O)ⱼR⁴_{f}
   wherein R³_{f} and R⁴_{f}, the same or different from each other, represent -CF₃ or -C₂F₅ and j is an integer.

The average molecular weight of perfluoropolyethers suitable for use in the instant invention generally is within the range of from about 500 to about 20,000. Preferably, their average molecular weight ranges from 1500 to 10,000.

The concentration of perfluoropolyethers in the cleansing emulsions is within the range of from 0.01 to 20% by weight, preferably from 0.05 to 5%.

The interface tension of the surfactants suitable for use in the process according to the present invention with the perfluoropolyether generally is lower than, or equal to, about 15 dyne.cm⁻¹, preferably lower than, or equal to, about 12 dyne.cm⁻¹.

Examples of suitable surfactants are: coco-amphoglycinate, coco-amido-betaine, lauroyl sarcosinate, DEA oleylamide (a mixture of diethanolamides and oleic acid), DEA coco-amide (a mixture of diethanolamides and coconut fatty acids), polyethyleneglycol-6-caprylic-capric triglycerides, dodecylamine oxide, polyethyleneglycol-7 coco acid monoglycerides and diglycerides, polyethyleneglycol-78-coco-mono- and di-glycerides, sodium laurylether sulfate-2.5 ethylene oxide and stearyl-dimethyl-benzyl-ammonium chloride.

With respect to the preparation process referred to above, the mixture of surfactant(s) with water is heated to a selected temperature within the range of those temperatures which are commonly used in the preparation of cleansing emulsions, i.e., of from about 25°C up to about 75°C, and preferably of from about 40°C up to about 75°C.

The mixture of thickeners (for viscosity purposes) and optional foam supporting substances with water is also heated up to a temperature within the range of temperatures commonly used in the preparation of cleansing emulsions, i.e., temperatures within the range of from about 25°C up to about 75°C, preferably of from about 40°C up to about 75°C. The viscosity of said mixture is adjusted by means of the addition of water, until a medium-viscosity solution is obtained. For that purpose, a viscosity usually comprised within the range of from about 500 to about 5000 centipoises (as measured at 25°C), and preferably of from about 1000 to about 2000 centipoises, is suitable.

The addition of the perfluoropolyether to either of the above mixtures is carried out with strong stirring. For that purpose, an apparatus is used, such as, e g., a turbine stirrer, which runs at a revolution speed of at least 3000 rpm, and preferably of at least 5000 rpm.

The perfluoropolyether preferably is added to the mixture of thickening agents and optional foam supporting agents with water.

Any optional preservatives, dyes, perfumes, softeners, opacifiers and sequestering agents are preferably the last to be added, i.e., they are added to the mixture of substances selected from surfactants, oils, fats and polyalcohols with the thickeners, the optional foam supporting agents, the perfluoropolyether and water, before the emulsion is cooled.

The resulting cleansing emulsions may be used as shampoos, foam baths, cleansing milks, cleansing creams, bath oils, liquid soaps and similar compositions for skin and hair cleansing.

The above surfactants commonly are selected from products which are customarily used in the preparation of cleansing emulsions for skin and hair, which simultaneously show a low interface tension with the perfluoropolyether.

The thickeners, and all of the other components generally are selected from those products which are usually used in the preparation of cleansing emulsions for skin and hair.

Suitable thickeners are, e.g., xanthan gum, guar gum and PEG 6000 distearate.

The main advantages of the present invention can be summarized as follows:
- stable emulsions of the perfluoropolyether in the cleansing compositions for skin and hair may be obtained;
- after the washing, a reduction in the rebound effect on hair and on seborrheic skin is obtained;
- a normalization of the redistribution of sebum on skin of asteatosic subjects is obtained after the washing.

The following examples are merely given for illustrative purposes, and are not limitative to the present invention.

### EXAMPLE 1

For comparative purposes a shampoo not containing a perfluoropolyether was prepared. Said shampoo had the following composition by weight:

| | |
|---|---|
| A) alkylamidobetaine | 5.0% |
| B) alkylamidoamine N-oxide | 4.0% |
| C) coco-ampho-glycinate | 5.0% |
| D) sodium laurylether sulfate | 5.0% |
| E) ethoxylated coco glycerides | 4.0% |
| F) sodium chloride | 1.0% |
| G) polyethyleneglycol 6000 distearate | 3.0% |
| H) deionized water | q.s. up to 100% |
| I) preservatives, dyes, perfume | q.s. |

The shampoo was prepared as follows:
(1) (A), (C) and (D) were mixed together with a portion of (H); then (F) was added and the whole mixture was carefully deaerated;
(2) (B) and (E) were melted together with (G) and water of 75°C was added until a pourable solution was obtained;
(3) (2) was slowly added to (1), and the mixture thus obtained was deaerated;
(4) (I) was added;
(5) the obtained mass was cooled with simultaneous stirring.

### EXAMPLE 2

A shampoo was prepared which was identical to the shampoo of example 1, but which additionally contained 1% of a perfluoropolyether with perfluoroalkyl end groups. The perfluoropolyether was Fomblin® HC/04 by Montefluos S.p.A., having the formula: 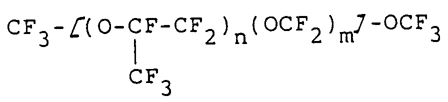 wherein n/m is within the range of from 20 to 40.

This perfluoropolyether has an average molecular weight of 1500 and a viscosity of 35 cSt at 20°C.

The shampoo had the following composition by weight:

| | |
|---|---|
| A) alkylamidobetaine | 5.0% |
| B) alkylamidoamine N-oxide | 4.0% |
| C) coco-ampho-glycinate | 5.0% |
| D) sodium laurylether sulfate | 5.0% |
| E) ethoxylated coco glycerides | 4.0% |
| F) sodium chloride | 1.0% |
| G) polyethyleneglycol 6000 distearate | 3.0% |
| H) perfluoropolyether Fomblin® HC/04 | 1.0% |
| I) deionized water | q.s. up to 100% |
| J) preservatives, dyes, perfume | q.s. |

The shampoo was prepared as follows:
(1) (A), (C) and (D) were mixed together with a portion of (I); then (F) was added and the whole mixture was carefully deaerated;
(2) (B) and (E) were melted together with (G) and water of 75°C was added until a pourable solution was obtained;
(3) (H) was dispersed in the mixture (2) by means of a turbine mixer;
(4) (3) was slowly added to (1), and the whole mixture was deaerated;
(5) (J) was added;
(6) the obtained mass was cooled with simultaneous stirring.

The shampoo according to the present invention, and the comparative shampoo, not containing perfluoropolyether, were evaluated as follows.

A preliminary study under the scanning electron microscope was carried out on seborrheic hair before and after the application of the shampoo which contained perfluoropolyether. Hair samples supplied by six seborrheic patients were studied. The hair samples were drawn from the same scalp region of all of said patients two days after the cleansing with the placebo shampoo which did not contain perfluoropolyether in its formulation (example 1).

A second sampling was carried out after one month of washings (twice a week) with the same shampoo containing 1% of perfluoropolyether (example 2).

Each hair was drawn by means of suitable tweezers and, without any preliminary treatments, was placed on a support on which it was fastened by means of a double-adhesive tape. Then the hair was metallized with 20 nm of gold-platinum on a Balzers MED 010 metallizer, and was observed by means of the Philips 505 scanning electron microscope.

The individual hairs were observed at their suprabulbar region.

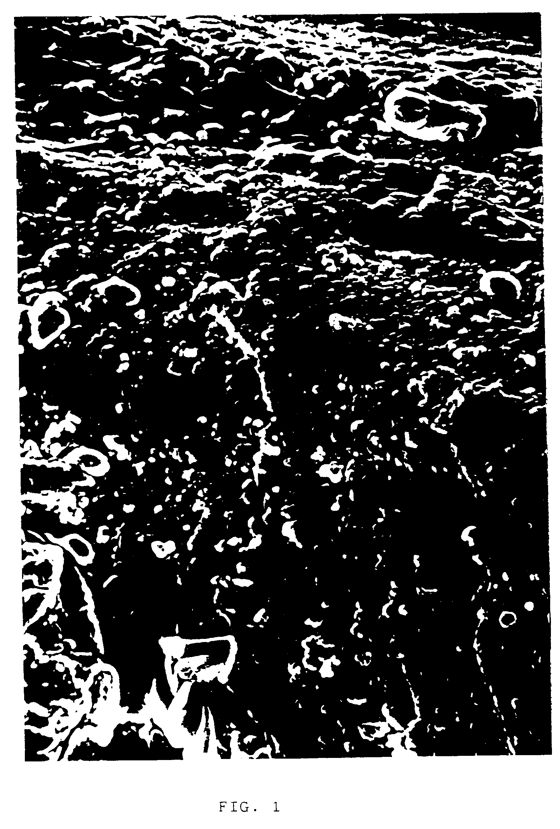

The observation was carried out at magnifications of from 1100 up to 8800. Under basal reference condition (see Figure 1, at 2100 X magnification), there was observed on the hair surface the presence of an "induitus" characterized by structurelacking polycyclic and semispherical masses or protrusions, which hindered, at least partially, the normal morphology of cuticular geometry.

These findings were identical with all of the analysed samples.

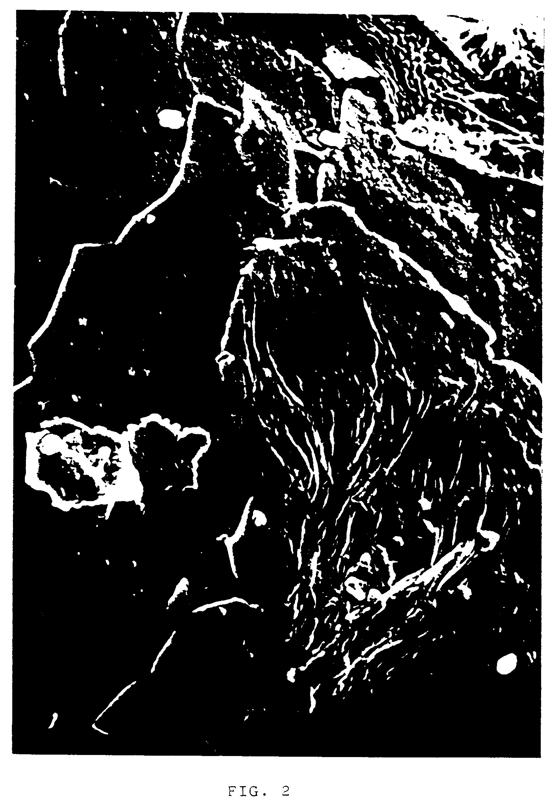

The observation of hairs after the treatment with the shampoo which contained perfluoropolyether (see Figure 2, at 2200 X magnification) revealed a significant decrease in the presence of said induitus which normally is to be attributed to the presence of sebum.

### EXAMPLE 3

A foam bath not containing perfluoropolyether was prepared for comparative purposes. Said foam bath had the following composition by weight:

| | |
|---|---|
| A) sodium laurylether sulfate | 15.0% |
| B) coco-ampho-glycinate | 8.0% |
| C) alkylamidobetaine | 10.0% |
| D) ethoxylated coco glycerides | 6.0% |
| E) alkylamidoamine N-oxide | 5.0% |
| F) polyethyleneglycol 6000 distearate | 2.0% |
| G) xanthan gum | 2.0% |
| H) sodium chloride | 1.0% |
| I) deionized water | q.s. up to 100% |
| J) preservatives, dyes, perfume | q.s. |

The foam bath was prepared as follows:
(1) (G) was dispersed in a portion of (I) and in (H) until a homogeneous gel was obtained; the latter was then deaerated, and (A), (B) and (C) were added under vacuum;
(2) (D), (E) and (F) were melted at 75°C and water of 75°C was added until a pourable solution was obtained;
(3) (2) was slowly added to (1) and the whole mixture was deaerated;
(4) (J) was added;
(5) the obtained mass was cooled with simultaneous stirring.

### EXAMPLE 4

A foam bath was prepared which was identical to the foam bath of example 3, but additionally contained 1% of a perfluoropolyether with perfluoroalkyl end groups. The perfluoropolyether was Fomblin® HC/R by Montefluos S.p.A., having the same formula as specified in example 2.

This perfluoropolyether had an average molecular weight of 6600 and a viscosity of 1500 cSt at 20°C.

The foam bath had the following composition, by weight:

| | |
|---|---|
| A) sodium laurylether sulfate | 15.0% |
| B) coco-ampho-glycinate | 8.0% |
| C) alkylamidobetaine | 10.0% |
| D) ethoxylated coco glycerides | 6.0% |
| E) alkylamidoamine N-oxide | 5.0% |
| F) polyethyleneglycol 6000 distearate | 2.0% |
| G) xanthan gum | 2.0% |
| H) sodium chloride | 1.0% |
| I) deionized water | q.s. up to 100% |
| J) perfluoropolyether Fomblin® HC/R | 1.0% |
| K) preservatives, dyes, sequestering agents, perfumes | q.s. |

The foam bath was prepared as follows:
(1) (G) was dispersed in a portion of (I) and in (H) until a homogeneous gel was obtained; the latter then was deaerated and (A), (B) and (C) were added under vacuum;
(2) (D), (E) and (F) were melted at 75°C and water of 75°C was added until a pourable solution was obtained;
(3) (J) was dispersed in (2) by means of a turbine stirrer;
(4) (3) was slowly added to (1) and the whole mixture was deaerated;
(5) the obtained mass was cooled with simultaneous stirring.

The foam bath according to the present invention (example 4) and the foam bath not containing perfluoropolyether (example 3) were evaluated as follows.

The sebacic response of skin to cleansing was determined in vivo. Thirty healthy volunteers, 13 to 81 years old, were selected.

At the objective examination, the volunteers' skins appeared to be normal or mixed in 39.6%, seborrheic in 11.4% and asteatosic in 49% of the cases. The skin regions subjected to evaluation were: the forehead (at glabella level) and the presternal region (immediately under the sternal angle).

On each subject, in both of these regions, two cleansing agents were used, which agents corresponded to the preparations disclosed in examples 3 and 4. The subjects with normal, mixed or seborrheic skin were told to use, always at the same time in the morning, for ten consecutive days, the perfluoropolyether-containing cleansing preparation on the forehead and the placebo on the presternal region; the subjects with asteatosic skin were told to do the contrary.

The determinations of the sebacic response after cleansing were carried out according to two different methods: with the Schwarzhaupt SM 410 sebometer, and with Sebumtape® adhesive tapes (Cuderm Co. Dallas, U.S.A.; Hermal Pharm. Lab., Oak Hill, U.S.A.).

Said determinations were carried out between 9 and 11 a.m., under constant conditions of room temperature and humidity (50% ± 2% of humidity, 22°C ± 1°C).

The sebometric measurements were carried out:
1) under basal conditions ("lipid casual level");
2) soon after a strong washing with 30% isopropanol (the sebometric index being practically set to zero);
3) one hour after the cleansing according to (2);
4) after 24 hours, with the cleansing emulsion being used 1 hour in advance;
5) after 10 days of daily use, in the morning, of the cleansing emulsions, 1 hour after the last washing.

The Sebumtape® strip was simultaneously applied on a skin region immediately adjacent to the region on which the sebometric measurement was carried out, was left on said region for 1 hour, was then removed, applied onto a piece of thin cardboard of black colour, and finally was photographed with a constant magnification.

The measurements carried out under basal conditions confirmed the distribution of the sebometric values by sex, age and body region.

The sebometric determinations carried out in order to quantify the response to the cleansing treatment afforded the following results:
- In the subjects with normal, mixed or seborrheic skin, the washing with isopropanol led to a considerable increase in sebometric value already after 1 hour.
   After 24 hours (1 hour after the use of the cleansing emulsion), the sebometric values had further increased.
   After 10 days of constant use of the cleansing emulsion containing the perfluoropolyether, the treated regions showed a lower average sebometric value than in the preceding measurement (i.e., the measurement carried out after 24 hours).
   The skin regions treated in the same way with the cleansing system not containing perfluoropolyether showed an increase in sebum (rebound effect).
- In the subjects with asteatosic skin, the average sebometric increase occurred more slowly and reached a maximum after 10 days of use of the cleansing agent with the perfluoropolyether. On the contrary, in skin regions treated with the placebo, the value remained constantly low.

The above results were confirmed by the inspection of the photographs of Sebumtapes®, in which a decrease was observed in number and in size of black spots (decrease in seborrhea) in the case of subjects with normal, mixed or seborrheic skin treated with perfluoropolyether-containing cleanser, contrary to what was observed on subjects with asteatosic skin.

## Claims

1. Cleansing emulsions suitable for shampoos, foam baths, liquid soaps and similar compositions for skin and hair cleansing, capable of reducing the redistribution of sebum on skin and hair and of normalizing the redistribution of sebum on skin and hair of asteatosic subjects, said cleansing emulsions consisting of:
1) a perfluoropolyether with perfluoroalkyl end groups in an amount of from 0.01 to 20% by weight;
2) at least one surfactant having a low interface tension with said perfluoropolyether;
3) one or more thickening agents;
4) water;
5) optionally, one or more foam supporting substances;
6) optionally, one or more softeners and/or perfumes and/or dyes and/or preservatives and/or opacifiers and/or sequestering agents.

2. Use of the cleansing emulsions according to claim 1 for shampoos, foam baths and liquid soaps.

## Patentansprüche

1. Reinigungsemulsionen, die sich für Shampoos, Schaumbäder, Flüssigseifen und ähnliche Zusammensetzungen für die Haut- und Haarreinigung eignen und die Neuverteilung von Sebum auf Haut und Haar vermindern und die Neuverteilung von Sebum auf Haut und Haar von Patienten mit Asteatosis normalisieren können, bestehend aus:
1) einem Perfluorpolyether mit Perfluoralkyl-Endgruppen in einer Menge von 0,01 bis 20 Gewichts-%;
2) mindestens einem Tensid mit niedriger Grenzflächenspannung mit dem Perfluorpolyether;
3) einem oder mehreren Verdickungsmitteln;
4) Wasser;
5) gegebenenfalls einer oder mehreren schaumstützenden Substanzen;
6) gegebenenfalls einem oder mehreren Weichmachern und/oder Parfums und/oder Farbstoffen und/oder Konservierungsmitteln und/oder trüb machenden Mitteln und/oder Sequestriermitteln.

2. Verwendung der Reinigungsemulsionen nach Anspruch 1 für Shampoos, Schaumbäder und Flüssigseifen.

## Revendications

1. Emulsions de nettoyage appropriées pour shampooings, bains moussants, savons liquides et compositions analogues pour le nettoyage de la peau et des cheveux, capables de réduire la redistribution de sébum sur la peau et les cheveux et de normaliser la redistribution de sébum sur la peau et les cheveux de sujets astéatosiques, lesdites émulsions de nettoyage consistant en :
(1) un perfluoropolyéther ayant des groupes terminaux perfluoroalkyle dans une quantité de 0,01 à 20% en poids ;
(2) au moins un agent tensio-actif ayant un faible tension d'interface avec ledit perfluoropolyéther ;
(3) un ou plusieurs agents épaississants ;
(4) de l'eau ;
(5) facultativement, une ou plusieurs substances de maintien de la mousse ;
(6) facultativement, un ou plusieurs émollients et/ou parfums et/ou colorants et/ou conservateurs et/ou opacifiants et/ou agents séquestrants.

2. Utilisation des émulsions de nettoyage telles que définies à la revendication 1, pour des shampooings, des bains moussants et des savons liquides.
